# EUROPÄISCHE PATENTSCHRIFT

(11) **EP 2 155 754 B1**
(45) Veröffentlichungstag und Bekanntmachung des Hinweises auf die Patenterteilung: **24.10.2012**
(21) Anmeldenummer: 08736494.9
(22) Anmeldetag: 23.04.2008
(51) Int. Cl.: C07D 493/04

(54) **VERFAHREN ZUR HERSTELLUNG VON DIANHYDROHEXITOL-DIESTERN**
METHOD FOR THE PRODUCTION OF DIANHYDROHEXITOL DIESTERS
PROCÉDÉ DE FABRICATION DE DIESTERS DE DIANHYDROHEXITOL

(30) Priorität: 21.06.2007 DE 102007028702
(43) Veröffentlichungstag der Anmeldung: 24.02.2010
(73) Patentinhaber: Evonik Oxeno GmbH, 45772 Marl (DE)
(72) Erfinder: GRASS, Michael, 45721 Haltern Am See (DE); WOELK-FAEHRMANN, Michael, 45768 Marl (DE)
(86) Internationale Anmeldenummer: PCT/EP2008/054916
(87) Internationale Veröffentlichungsnummer: WO 2008/155159

(56) Entgegenhaltungen:
- WO-A-96/25384
- WO-A-2006/103338

## Beschreibung

Die vorliegende Erfindung betrifft ein Verfahren zur Herstellung von Isosorbid-di-2-ethylhexanoat durch Veresterung von Isoidid mit 2-Ethylhexansäure in Gegenwart eines Katalysators.

Diester von Dianhydrohexitolen, insbesondere jene auf Basis von Isosorbid, wurden bereits verschiedentlich als geeignete Weichmacher für PVC beschrieben. Nicht zuletzt daher hat es nicht an Versuchen gefehlt, Verfahren zur Herstellung dieser Verbindungen zu entwickeln, die die gewünschten Produkte in hoher Reinheit und mit der für die Verwendung in transparenten Anwendungen unabdingbaren geringen Farbzahl, also möglichst wasserklar, liefern können.

Dianhydrohexitoldiester können durch Umsetzung von Hexitolen, Monoanhydrohexitolen oder bevorzugt Dianhydrohexitolen mit Carbonsäuren in Gegenwart eines sauren Katalysators unter Wasserabspaltung hergestellt werden, wie beispielsweise in GB 613 444, US 3 454 603 oder WO 99/045060 beschrieben. In WO 01/83488 wird ein Verfahren dargelegt, das keinen homogen gelösten Katalysator, sondern ein festes saures Ionenaustauscherharz als Katalysator verwendet, was die Aufarbeitung erleichtert.

Die beschriebenen Verfahren haben jedoch den Nachteil, dass sie zumindest in einem der Kriterien, nämlich Selektivität hinsichtlich des Diesters, Reaktionszeit oder Verfärbung nicht zufriedenstellend sind.

In WO 2006/203338 wird ein Verfahren zur Herstellung von Dianhydrohexitoldiestern beschrieben, welches durch die Verwendung einer Kombination zweier Katalysatoren, nämlich eines dort näher beschriebenen sauren Katalysators und Hypophosphoriger Säure (H₃PO₂), die gewünschten Diester in Selektivitäten größer 95 % und niedrigen Farbzahlen, ausgedrückt durch den Yellowness Index, bereitstellt.

Allerdings zeigt dieses Verfahren den Mangel, dass nur durch eine Kombination zweier Katalysatoren der gewünschte Effekt bezüglich hoher Reinheit und geringer Verfärbung erzielt werden kann. Auch aus Kostengründen wäre ein Verfahren, das mit nur einem Katalysator durchgeführt werden kann und trotzdem die oben genannten Kriterien erfüllt, erstrebenswert. Darüber hinaus wäre es wünschenswert, Produkte mit noch geringerer Farbzahl zu erhalten.

Völlig überraschend wurde gefunden, dass die Veresterung von Isosorbid mit 2-Ethylhexansäure in Gegenwart von H₃PO₂ als alleinigem Katalysator mindestens gleichwertige Resultate liefert wie die in WO 2006/203338 beschriebene Prozedur. Dies bezieht sich insbesondere auf erzielbare Farbzahlen und Selektivitäten hinsichtlich der Bildung von Isosorbiddiestern.

Gegenstand der vorliegenden Erfindung ist daher ein Verfahren zur Herstellung von Isosorbid-di-2-ethylhexanoat umfassend die Verfahrensschritte:
a) Veresterung von Isosorbid mit 2-Ethylhexansäure mittels saurem Katalysator,
b) Abdestillieren der überschüssigen 2-Ethylhexansäure,
c) Reduzierung der Säurezahl mittels basischem Aluminiumoxids,
d) Filtration,
wobei als Katalysator im Verfahrensschritt a) ausschließlich Hypophosphorige Säure eingesetzt wird.

Das Isosorbid kann vor dem Einsatz in dem erfindungsgemäßen Verfahren durch jedes an sich bekannte Verfahren zur Dehydratisierung eines Hexitols oder Hexitol-Gemischs hergestellt worden sein, an das sich nach der Neutralisation üblicherweise zumindest eine Aufreinigung des entstandenen Reaktionsprodukts anschließt.

Die Aufreinigungsstufe kann aus einer einfachen Destillation des aus der Dehydratisierung hervorgegangenen Mediums bestehen. Dabei erhält man die Dianhydrohexitol-Verbindung in Form eines Rohdestillats, als Isosorbid-Rohdestillat.

Dieses Destillat kann zumindest eine weitere Aufreinigungsstufe durchlaufen, insbesondere eine Aufreinigung durch Kristallisation in einer wässerigen Phase oder in einer Lösemittelphase, durch Aufkonzentrierung im Vakuum und/oder durch eine ein- oder mehrfache Behandlung mit lonentauscherharz und/oder Aktivkohle in Pulver- und/oder Granulatform, wie etwa in WO 01/94352 beschrieben.

Unabhängig davon, ob nach der Destillation eine Aufreinigung erfolgt oder nicht, weist das als Rohstoff bei dem Verfahren gemäß der Erfindung verwendete Ausgangsprodukt vorteilhafterweise einen hohen Dianhydrohexitol-Anteil von mindestens 95 Massen-%, vorzugsweise von mindestens 98 Massen-% und besonders bevorzugt von mindestens 98,5 Massen-% auf, wobei sich diese Prozentangaben auf das gesamte Trockengewicht des Dianhydrohexitols im Verhältnis zum Trockengewicht des Ausgangsprodukts beziehen.

In dem erfindungsgemäßen Verfahren wird Isosorbid oder ein Produkt, welches mindestens 95 Massen-% Isosorbid aufweist, mit 2-Ethylhexansäure in Gegenwart von Hypophosphoriger Säure verestert. Die zur Bildung des Esters eingesetzte 2-Ethylhexansäure wird vorzugsweise im Überschuss, bevorzugt mit einem molaren Überschuss von 5 bis 50 %, insbesondere 10 bis 30 % der zur Bildung des Diesters notwendigen molaren Menge eingesetzt.
Zur Entfernung des bei der Veresterung entstehenden Reaktionswassers kann es vorteilhaft sein, wenn dieses mit der 2-Ethylhexansäure aus dem Reaktionsgemisch abdestilliert wird. Die 2-Ethylhexansäure dient somit als Schleppmittel.
Die Abdestillation von Reaktionswasser und gegebenenfalls Schleppmittel kann auch als Vakuumdestillation bei entsprechend reduziertem Druck durchgeführt werden. Optional kann auch ein anderes Schleppmittel, beispielsweise Toluol, Benzol, Cyclohexan, Hexan, Heptan oder Xylole eingesetzt werden. Dies kann insbesondere dann vorteilhaft sein, wenn beispielsweise das Vakuum nicht so einstellbar ist, dass das Reaktionswasser als Gemisch mit der 2-Ethylhexansäure ausreichend schnell abdestilliert.
Alternativ zu oder in Kombination zu dem reduzierten Druck, kann das bei der Veresterung entstehende Reaktionswasser auch durch Durchleiten eines inerten Gases abgetrennt werden.

Die zur effektiven Abtrennung des Reaktionswassers einzustellenden Bedingungen bezüglich Druck und Inertgasstrom sind dem Fachmann geläufig und können leicht durch Vorversuche ermittelt werden.
Die durch Aufarbeitung des Destillats gewonnene 2-Ethylhexansäure kann teilweise oder vollständig in die Reaktion zurückgeführt werden. Es ist auch möglich, die Aufarbeitung des Destillats zu einem späteren Zeitpunkt durchzuführen und die entfernte Flüssigkeitsmenge ganz oder teilweise durch frische 2-Ethylhexansäure, d. h. aus einem Vorratsgefäß, zu ersetzen.

Die in dem erfindungsgemäßen Verfahren zur Herstellung von Isosorbid-di-2-ethylhexanoat durch sauer katalysierte Veresterung von Isosorbid als saurer Katalysator ausschließlich und einzig einzusetzende hypophosphorige Säure wird bevorzugt in Form einer wässrigen Lösung angewendet. Die hypophosphorige Säure kann dabei in beliebigen Verdünnungen eingesetzt werden. Besonders bevorzugt sind Konzentrationen von 30 bis 60 Massen-%, insbesondere 40 bis 50 Massen-%. Bezogen auf die Einwaage an Dianhydrohexitol werden 0,01 bis 5 % Hypophosphorige Säure (gerechnet als Reinstoff), bevorzugt 0,03 bis 1 %, besonders bevorzugt 0,05 bis 0,5 % verwendet. Der Katalysator kann vor oder während der Veresterung zugesetzt werden, vorzugsweise bereits beim Aufheizen.

Die Veresterungstemperatur kann in Abhängigkeit von der verwendeten Säure oder der Empfindlichkeit der Diester 100 bis 260 °C, bevorzugt 150 bis 260 °C betragen. In der Regel arbeitet man bei Bedingungen, bei denen das Gemisch aus Wasser und 2-Ethylhexansäure siedet.

Die Rohestergemische, die neben dem Esterprodukt noch gewisse Mengen an den Ausgangsproduktenlsosorbid, 2-Ethylhexansäure, Katalysator und/oder deren Folgeprodukte sowie gegebenenfalls Nebenprodukte enthalten, können nach an sich bekannten Verfahren aufgearbeitet werden. Die Aufarbeitung umfasst dabei vorzugsweise folgende Schritte: Abtrennung der überschüssigen 2-Ethylhexansäure und gegebenenfalls Leichtsieder durch Destillation, gegebenenfalls in Anwesenheit von Aktivkohle, Neutralisation der vorhandenen Säuren, optional eine Wasserdampfdestillation, Waschen des Rohproduktes, und Trocknung sowie

Abtrennung restlicher Feststoffe mittels Filtration.
Im Regelfall sind Maßnahmen zur Farbverbesserung nötig, wie zum Beispiel Rühren mit Aktivkohle, Entfärben durch Einsatz von polymeren Adsorbentien oder auch Bleichen mit beispielsweise Wasserstoffperoxid-Lösung. Dabei können je nach angewendetem Aufarbeitungsverfahren die Reihenfolge dieser Schritte verschieden sein. Die vorgenannte Neutralisation kann auch stufenweise durchgeführt werden, indem zunächst mit einer schwachen Base (zum Beispiel Natriumcarbonat- oder Natriumhydrogencarbonat-Lösung) die stärkere Säure H₃PO₂ und erst im Anschluss an das Abdestillieren noch verbliebene Reste der Carbonsäure durch Neutralisation mit Natronlauge oder Kalilauge oder anderen starken basischen Lösungen entfernt werden.
Es hat sich gezeigt, dass es im Hinblick auf eine Farbverbesserung vorteilhaft ist, einen Waschschritt durchzuführen, bei dem wasserlösliche Verunreinigungen extrahiert und über eine anschließende Phasentrennung entfernt werden. Ohne einen solchen Schritt besteht die Möglichkeit, dass sich bei der zur Trocknung nötigen erhöhten Temperatur der Ansatz wieder verfärbt.
In einer besonders bevorzugten Ausführungsform des Verfahrens kann nach Abdestillieren des Überschusses an 2-Ethylhexansäure eine
Wasserdampfdestillation, bevorzugt in Gegenwart von Aktivkohle, durchgeführt werden, wodurch sowohl der Katalysator als auch die 2-Ethylhexansäure so weit abgetrennt werden können, dass eine Neutralisation und eventuell nachfolgende Waschschritte zur Abtrennung der hierdurch gebildeten Verbindungen unterbleiben können. Wenn die Farb- und Säurezahl des Produktes danach zufrieden stellend ist, kann auf eine weitere Aufarbeitung verzichtet werden. Diese Variante bietet sich besonders dann an, wenn von hochreinen und farblosen Edukten, insbesondere von einer hohen Isosorbid-Qualität ausgegangen wird. In Beispiel 3 wird eine solche Ausführungsform näher beschrieben.

Die Bestimmung der Farbzahlen während der Reaktion oder während der Aufarbeitungsschritte erfolgt nach an sich bekannter Prozedur mit einem handelsüblichen Farbzahl-Messgerät.

Die erfindungsgemäß hergestellten Ester des Isosorbids zeichnen sich durch eine besonders hohe Reinheit und geringe Verfärbung aus und zeigen äußerst niedrige Farbzahlwerte. Die als Veresterungskatalysator eingesetzte Hypophosphorige Säure lässt sich praktisch rückstandsfrei abtrennen. Der analytisch nachweisbare Restphosphorgehalt liegt in der ppm-Größenordnung.

Die erfindungsgemäß hergestellten Ester des Isosorbids, können in Farben, Tinten oder Lacken, in Plastisolen, Klebstoffen oder Klebstoffkomponenten, in Dichtungsmassen, als Weichmacher in Kunststoffen oder Kunststoffkomponenten, als Lösemittel, als Schmierölkomponente, als Kühlflüssigkeit oder Bohrflüssigkeit oder Bestandteil davon oder als Hilfsmittel bei der Metallverarbeitung verwendet werden. Bevorzugte Plastisole sind dabei insbesondere PVC- oder PAMA-Plastisole. Bevorzugte Kunststoffe sind insbesondere Polyvinylchlorid (PVC), Polyvinylbutyral (PVB), Homo- und Copolymere auf Basis von Ethylen, Propylen, Butadien, Vinylacetat, Celluloseacetat, Glycidylacrylat, Glycidylmethacrylat, Methacrylaten, Acrylaten, Acrylaten mit am Sauerstoffatom der Estergruppe gebundenen Alkylresten von verzweigten oder unverzweigten Alkoholen mit einem bis zehn Kohlenstoffatomen, Styrol, Acrylnitril, Homo- oder Copolymere von cyclischen Olefinen.

Als Vertreter der obigen Gruppen seien beispielsweise folgende Kunststoffe genannt: Polyacrylate mit gleichen oder verschiedenen Alkylresten mit 4 bis 8 C-Atomen, gebunden am Sauerstoffatom der Estergruppe, insbesondere mit dem n-Butyl-, n-Hexyl-, n-Octyl- und 2-Ethylhexylrest, Polymethacrylat, Polymethylmethacrylat, Methylacrylat-Butylacrylat-Copolymere, Methylmethacrylat-Butylmethacrylat-Copolymere oder allgemein Polyalkylmethacrylate (PAMA), Ethylen-VinylacetatCopolymere, chloriertes Polyethylen, Nitrilkautschuk, Acrylnitril-Butadien-StyrolCopolymere, Ethylen-Propylen-Copolymere, Ethylen-Propylen-Dien-Copolymere, Styrol-Acrylnitril-Copolymere, Acrylnitril-Butadien-Kautschuk, Styrol-Butadien-Elastomere, Methylmethacrylat-Styrol-Butadien-Copolymere, Celluloseacetat, PVB und PVC. Ein besonders bevorzugter Kunststoff ist dabei PVC.

Darüber hinaus können die erfindungsgemäß hergestellten Produkte zur Modifizierung von Kunststoffmischungen, beispielsweise der Mischung eines Polyolefins mit einem Polyamid, eingesetzt werden.

Zusammensetzungen aus Kunststoff/-en, insbesondere PVC oder PAMA, die die erfindungsgemäß hergestellten Produkte enthalten, können beispielsweise in folgenden Produkten enthalten sein: Gehäuse für Elektrogeräte, wie beispielsweise Küchengeräte, Computergehäuse, Gehäuse und Bauteile von Phono- und Fernsehgeräte, Rohrleitungen, Apparate, Kabel, Drahtummantelungen, Isolierbänder, im Innenausbau, im Fahrzeug- und Möbelbau, Plastisole, in Bodenbelägen, medizinische Artikel, Lebensmittelverpackungen, Dichtungen, Folien, Verbundfolien, Schallplatten, Kunstleder, Spielzeug, Verpackungsbehälter, Klebebandfolien, Bekleidung, Beschichtungen, Beflockungen und Bedruckungen, Fasern für Gewebe, beschichtete Gewebe.
Weiterhin können Zusammensetzungen aus Kunststoff, insbesondere PVC, die erfindungsgemäß hergestellte Produkte enthalten, beispielsweise zur Herstellung folgender Erzeugnisse verwendet werden: Rohrleitungen, Schläuche, Kabel, DrahtUmmantelungen, Isolierbänder, im Fahrzeug- und Möbelbau, Plastisole, Profile, Bodenbeläge, medizinische Artikel (wie z. B. Blutbeutel, Schläuche, Infusionsbeutel etc.), Spielzeuge, Lebensmittelverpackungen, Dichtungen, Folien, Verbundfolien, Platten, Kunstleder, Tapeten, Verpackungsbehälter, Klebebandfolien, Bekleidung, Beschichtungen oder Fasern für Gewebe, Schuhe, Unterbodenschutz, Nahtabdichtungen, Dachbahnen, Modelliermassen oder Bälle.

PVC-Zusammensetzungen oder Plastisole, die PVC und erfindungsgemäß hergestellte Produkte enthalten, enthalten vorzugsweise von 5 bis 250 Massenteile, bevorzugt von 10 bis 200 Massenteile und besonders bevorzugt von 20 bis 100 Massenteile an den erfindungsgemäßen Produkten pro 100 Massenteilen PVC.

Die folgenden Beispiele sollen die Erfindung erläutern, ohne deren Anwendungsbreite einzuschränken, die sich aus der Beschreibung und den Patentansprüchen ergibt.

### Beispiele:

### Beispiel 1: Herstellung von Isosorbid-di-2-ethylhexanoat (Vergleichsbeispiel)

In einem 1-Liter-Mehrhalskolben mit Rührer, Wasserabscheider, Rückflusskühler, Tropftrichter und Innenthermometer wurden 146 g (1 Mol) Isosorbid (Fa. Cerestar) mit 438 g (3 Mol) 2-Ethylhexansäure (Fa. Sigma Aldrich) und 0,88 g einer 50%igen wässrigen Lösung von Hypophosphoriger Säure (Fa. Sigma Aldrich) (0,3 Massen-% H₃PO₂ bezogen auf Isosorbid) unter Rühren auf 240 °C erhitzt. Durch Anlegen eines leichten Vakuums wurde bei dieser Temperatur gewährleistet, dass das Reaktionswasser über den Wasserabscheider komplett ausgetragen werden konnte. Der Reaktionsverlauf wurde über GC-Analytik verfolgt. 7 Stunden nach Siedebeginn war die Reaktion beendet (1. Farbzahlmessung) und der Wasserabscheider wurde durch eine Destillationsbrücke ersetzt, über die bei einer Temperatur bis 200 °C die überschüssige 2-Ethylhexansäure abdestilliert werden konnte. Hierbei wurde sukzessive der Druck bis etwa 2 hPa reduziert. Anschließend wurde der Ansatz auf 90 °C abgekühlt und mit 8,55g (2,5 Massen-% bezogen auf Kolbeninhalt) Wasserstoffperoxid-Lösung (35%ig, Fa. Merck) versetzt und eine Stunde bei konstanter Temperatur gerührt. Anschließend wurde das überschüssige Wasserstoffperoxid und Wasser abdestilliert (125 °C, 5 hPa) (2. Farbzahlmessung). Der Ansatz wurde danach auf 80 °C abgekühlt und mit 50 ml einer 3%igen NaOH-Lösung neutralisiert. Anschließend wurden weitere 80 ml einer 5%igen NaCl Lösung zugegeben und bei 90 °C noch weitere 30 Minuten gerührt. Die organische Phase wurde dann abgetrennt und nochmals mit 80 ml 5%iger NaCl Lösung 30 Minuten bei 90 °C gerührt und die wässrige Phase wiederum abgelassen. Danach wurde bei 125 °C unter Vakuum (2 hPa) getrocknet. Dann wurde der Ansatz nochmals mit 2 Massen-% Aktivkohle gerührt und danach filtriert (3. Farbzahlmessung). Der Gehalt an Isosorbiddiester, ermittelt durch GC lag bei 99 %.

Die Farbzahlen, ermittelt mit dem Farbzahl-Messgerät LICO 400 (Firma Hach-Lange, 11 mm Rundküvette), sind im Anschluss an Beispiel 2 in Tabelle 1 aufgeführt. Dazu wurde dem Reaktionsansatz mit einer Pipette eine Probe von ca. 20 ml entnommen, mit Hilfe eines Spritzenfilters filtriert und in die Messküvette des Farbzahl-Messgerätes gefüllt. Vor Aufnahme der Messungen wurde das Gerät mit vollentsalztem Wasser kalibriert. Gemessen wurden jeweils die Farbzahl nach Hazen (APHA) und die lod-Farbzahl.

### Beispiel 2: Herstellung von Isosorbid-di-2-ethylhexanoat (Vergleichsbeispiel)

In Anlehnung an Beispiel 2, Versuch 6 aus WO 2006/203338, wurden in einem 1-Liter-Mehrhalskolben, ausgestattet mit Rührer, Wasserabscheider, Rückflusskühler, Tropftrichter und Innenthermometer 146 g (1 Mol) Isosorbid (Fa. Cerestar) mit 438 g (3 Mol) 2-Ethylhexansäure (Fa. Sigma Aldrich), 2,92 g para-Toluolsulfonsäure-Monohydrat (2 Massen-% bezogen auf Isosorbid) und 0,88 g einer 50%igen wässrigen Lösung von Hypophosphoriger Säure (Fa. Sigma Aldrich) (0,3 Massen-% H₃PO₂ bezogen auf Isosorbid) unter Rühren bei einer Temperatur von 160 bis 175 °C erhitzt. Durch Anlegen eines leichten Vakuums wurde bei dieser Temperatur gewährleistet, dass das Reaktionswasser über den Wasserabscheider komplett ausgetragen werden konnte. Der Reaktionsverlauf wurde über GC-Analytik verfolgt. 7 Stunden nach Siedebeginn war die Reaktion beendet (1. Farbzahlmessung). Anschließend wurde der Ansatz bei 100 °C durch Zugabe einer Lösung von 1,8 g Na₂CO₃ in 6 g Wasser neutralisiert und dann, nach Aufheizen auf 200 °C, die 2-Ethylhexansäure im Vakuum abdestilliert. Anschließend wurde der Ansatz wieder auf 90 °C abgekühlt, mit 2,5 Massen-% H₂O₂ (35%ig, Fa. Merck) versetzt und eine Stunde bei konstanter Temperatur gerührt. Anschließend wurde das Wasserstoffperoxid / Wasser abdestilliert (125 °C, 5hPa) (2. Farbzahlmessung). Der Ansatz wurde auf 80 °C abgekühlt und mit 35 ml einer 3%igen NaOH-Lösung neutralisiert. Anschließend wurden weitere 40 ml einer 5% igen NaCl Lösung zugegeben und bei 90 °C noch weitere 30 Minuten gerührt. Die organische Phase wurde dann abgetrennt und nochmals mit 40 ml einer 5%igen NaCl Lösung 30 Minuten bei 90 °C gerührt und danach die wässrige Phase abgelassen. Darauf wurde bei 125 °C unter Vakuum (2 hPa) getrocknet. Danach wurde der Ansatz nochmals mit 2 Massen-% Aktivkohle (Actec PC 500) gerührt und sodann filtriert (3. Farbzahlmessung). Der Gehalt an Isosorbiddiester betrug ebenfalls 99 %.

**Tabelle 1: Übersicht über die erzielten Farbzahlen, jeweils gemessen in Anlehnung an DIN ISO 6271**

| | **Ende Veresterung Messung 1** | **Farbe nach Bleichen mit H₂O₂ Messung 2** | **Farbe nach Rühren mit A-Kohle Messung 3** |
|---|---|---|---|
| **Beispiel 1 (Vergleichsbeispiel)** | | | |
| APHA | 236 | 138 | 30 |
| Iod | 1,3 | 0,8 | 0,2 |
| **Beispiel 2 (Vergleichsbeispiel)** | | | |
| APHA | 553 | 368 | 58 |
| Iod | 2 | 2 | 0,2 |

| | | | |
|---|---|---|---|
| (Zwecks besserer Vergleichbarkeit wurden alle Messungen in einer 11 mm-Rundküvette durchgeführt, auch wenn bei niedrigen Farbzahlen laut DIN ISO 6271 eine 50 mm Küvette vorgeschlagen wird.) | | | |

### Beispiel 3: Herstellung von Isosorbid-di-2-ethylhexanoat mit optimierter Aufarbeitung (erfindungsgemäß)

In einem 4-Liter-Destillationskolben mit Rührstäbchen, Wasserabscheider, Rückflusskühler, Tropftrichter und Innenthermometer wurden 876 g (6 Mol) Isosorbid (Polysorb P, Fa. Roquette Frères) mit 2190 g (15 Mol) 2-Ethylhexansäure (Fa. Sigma Aldrich) und 1,75 g einer 50%igen wässrigen Lösung von Hypophosphoriger Säure (Fa. Sigma Aldrich) (0,2 Massen-% H₃PO₂ bezogen auf Isosorbid) unter Rühren auf 240 °C erhitzt. Durch Anlegen eines leichten Vakuums wurde bei dieser Temperatur gewährleistet, dass das Reaktionswasser über den Wasserabscheider komplett ausgetragen werden konnte. Der Reaktionsverlauf wurde über GC-Analytik verfolgt. 12,5 Stunden nach Siedebeginn war die Reaktion beendet (Hazen-Farbzahl in 50mm Küvette nach Abkühlen APHA = 41). Von diesem Ansatz wurden 750 g entnommen, in eine entsprechend kleinere Apparatur, bei der der Wasserabscheider durch eine Destillationsbrücke ersetzt war, gegeben und mit 1 Massen-% Aktiv-Kohle vom Typ A-Supra EUR (Fa. Norit) versetzt und auf ein Vakuum von 27 hPa eingestellt. Unter Rühren wurde bis zu bei einer Temperatur von 240 °C die überschüssige 2-Ethylhexansäure abdestilliert. Anschließend wurde die Heizung ausgeschaltet und der Ansatz durch tropfenweise Zugabe von 40 g Wasser über den Tropftrichter bei einem Druck von 27 bis 33 hPa auf 140 °C abgekühlt, wodurch eine weitere Reinigung des Ansatzes (Prinzip Wasserdampfdestillation) erfolgte. Danach wurde mittels Filtration das Produkt von der Aktivkohle abgetrennt und eine Probe entnommen (Farbzahl nach DIN ISO 6271: APHA 13, Säurezahl nach DIN EN ISO 2114 0,11 mg KOH/g). Zur weiteren Reduzierung der Säurezahl wurde mit 2 % basischem Aluminiumoxid (Fa. Sigma Aldrich) noch eine Stunde bei einer Temperatur von 100 °C gerührt.
Nach Filtration zeigte das Produkt folgende Werte:
Säurezahl nach DIN EN ISO 2114: 0,008 mg KOH/g
Farbzahl nach DIN ISO 6271: APHA = 8

Gehalt an P: < 10 ppm

Gehalt an P, bestimmt mittels Mikrowellenaufschluss der organischen Matrix zur Elementbestimmung mittels ICP-OES nach DIN EN ISO 11885:
Die Bestimmung der P-Konzentration erfolgt in wässriger, salpetersaurer Lösung. Die organische Matrix wird mittels Mikrowellenaufschluss unter Druck mit Salpetersäure zerstört.
Gerät: Mikrowelle Ultra Clave III, Firma MLS
Reagenzien: Salpetersäure, 65%ig; VE-Wasser

Durchführung:
0,4g werden auf der Analysenwaage in ein 15 ml Quarzgefäß eingewogen und mit 6 ml Salpetersäure und 3 ml VE-Wasser versetzt. Nach Einbau in den Autoklaven der Mikrowelle erfolgt der Aufschluss automatisch nach Druck- und Temperaturkontrolle.

| | |
|---|---|
| Aufschlusszeit: | 1:30 h |
| max. Druck: | 90 bar |
| max. Temperatur: | 230 °C |

Nach erfolgtem Aufschluss wird der Inhalt der Quarzgefäße auf einen 25 ml Messkolben mit VE-Wasser überführt und aufgefüllt. Die anschließende Messung auf Phosphor erfolgt nach DIN EN ISO 11885 am ICP - OES Spektrometer bei den Wellenlängen 177,440 nm und 214,914 nm.

## Patentansprüche

1. Verfahren zur Herstellung von Isosorbid-di-2-ethylhexanoat umfassend die Verfahrensschritte:
a) Veresterung von Isosorbid mit 2-Ethylhexansäure mittels saurem Katalysator,
b) Abdestillieren der überschüssigen 2-Ethylhexansäure,
c) Reduzierung der Säurezahl mittels basischem Aluminiumoxids,
d) Filtration,
wobei als Katalysator im Verfahrensschritt a) ausschließlich Hypophosphorige Säure eingesetzt wird.

2. Verfahren nach Anspruch 1,
**dadurch gekennzeichnet,**
**dass** die 2-Ethylhexansäure in einem molaren Überschuss von 5 bis 50 %, insbesondere 10 bis 30 % der zur Bildung des Diesters notwendigen molaren Menge eingesetzt werden.

3. Verfahren nach den Ansprüchen 1 bis 2,
**dadurch gekennzeichnet,**
**dass** bezogen auf die Einwaage an Isosorbid 0,01 bis 5 % Hypophosphorige Säure (gerechnet als Reinstoff), bevorzugt 0,03 bis 1 %, besonders bevorzugt 0,05 bis 0,5 % eingesetzt werden.

4. Verfahren nach den Ansprüchen 1 bis 3,
**dadurch gekennzeichnet,**
**dass** die einzusetzende Hypophosphorige Säure in Form einer wässrigen Lösung, vorzugsweise in einer Konzentration von 30 bis 60 Massen-%, insbesondere 40 bis 50 Massen-%, angewendet wird.

5. Verfahren nach den Ansprüchen 1 bis 4,
**dadurch gekennzeichnet,**
**dass** bei der Aufarbeitung der erhaltenen Isosorbiddiester zur Farbverbesserung eine Behandlung mit Aktivkohle und/oder Wasserstoffperoxid erfolgt.

6. Verfahren nach den Ansprüchen 1 bis 5,
**dadurch gekennzeichnet,**
**dass** das bei der Veresterung entstehenden Reaktionswassers mit der 2-Ethylhexansäure als Schleppmittel aus dem Reaktionsgemisch abdestilliert wird.

7. Verfahren nach den Ansprüchen 1 bis 6,
**dadurch gekennzeichnet,**
**dass** die durch Aufarbeitung des Destillats gewonnene 2-Ethylhexansäure teilweise oder vollständig in die Reaktion zurückgeführt wird.

## Claims

1. A process for the preparation of isosorbide di-2-ethylhexanoate comprising the steps of:
a) Esterification of isosorbide with 2-ethylhexanoic acid by means of acidic catalyst,
b) Distillation to remove the excess 2-ethylhexanoic acid,
c) Reduction of the acid number by means of basic aluminium oxide,
d) Filtration,
hypophosphorous acid exclusively is used as catalyst in step a).

2. A process according to claim 1,
**characterized in that**
the 2-ethylhexanoic acid is used in a molar excess of from 5 to 50%, in particular from 10 to 30%, of the molar amount needed for formation of the diester.

3. A process according to either of claims 1 and 2,
**characterized in that**,
based on the starting weight of isosorbide, the amount used of hypophosphorous acid (calculated as pure substance) is from 0.01 to 5%, preferably from 0.03 to 1%, particularly preferably from 0.05 to 0.5%.

4. A process according to any of claims 1 to 3,
**characterized in that**
the hypophosphorous acid to be used is used in the form of an aqueous solution, preferably at a concentration of from 30 to 60% by weight, in particular from 40 to 50% by weight.

5. A process according to any of claims 1 to 4,
**characterized in that**
during the work-up of the resultant isosorbide diesters a treatment with activated charcoal and/or hydrogen peroxide takes place to improve colour.

6. A process according to any of claims 1 to 5,
**characterized in that**
the water of reaction produced during the esterification reaction is removed from the reaction mixture by distillation with the 2-ethylhexanoic acid as entrainer.

7. A process according to any of claims 1 to 6,
**characterized in that**
some or all of the 2-ethylhexanoic acid obtained via work-up of the distillate is returned to the reaction.

## Revendications

1. Procédé pour la préparation de di-2-éthylhexanoate d'isosorbide, comprenant les étapes de procédé :
a) estérification d'isosorbide avec de l'acide 2-éthylhexanoïque au moyen d'un catalyseur acide,
b) élimination par distillation de l'acide 2-éthylhexanoïque en excès,
c) réduction de l'indice d'acide au moyen d'un oxyde d'aluminium basique,
d) filtration,
de l'acide hypophosphoreux étant exclusivement utilisé comme catalyseur dans l'étape de procédé a) .

2. Procédé selon la revendication 1, **caractérisé en ce que** l'acide 2-éthylhexanoïque est utilisé en un excès molaire de 5 à 50%, en particulier de 10 à 30% par rapport à la quantité molaire nécessaire pour la formation du diester.

3. Procédé selon les revendications 1 et 2, **caractérisé en ce que**, par rapport à la pesée d'isosorbide, on utilise 0,01 à 5% d'acide hypophosphoreux (calculé sous forme de substance pure), de préférence 0,03 à 1%, de manière particulièrement préférée 0,05 à 0,5%.

4. Procédé selon les revendications 1 à 3, **caractérisé en ce que** l'acide hypophosphoreux à utiliser est utilisé sous forme d'une solution aqueuse, de préférence en une concentration de 30 à 60% en masse, en particulier de 40 à 50% en masse.

5. Procédé selon les revendications 1 à 4, **caractérisé en ce que** lors du traitement du diester d'isosorbide obtenu, pour améliorer la couleur, on réalise un traitement au charbon actif et/ou au peroxyde d'hydrogène.

6. Procédé selon les revendications 1 à 5, **caractérisé en ce que** l'eau de réaction formée lors de l'estérification est éliminée par distillation du mélange réactionnel avec l'acide 2-éthylhexanoïque comme agent d'entraînement.

7. Procédé selon les revendications 1 à 6, **caractérisé en ce que** l'acide 2-éthylhexanoïque obtenu par traitement du distillat peut être recyclé en partie ou complètement dans la réaction.
